# EUROPÄISCHE PATENTSCHRIFT

(11) **EP 3 481 454 B1**
(45) Veröffentlichungstag und Bekanntmachung des Hinweises auf die Patenterteilung: **10.09.2025**
(21) Anmeldenummer: 18759848.7
(22) Anmeldetag: 17.07.2018
(51) Int. Cl.: A61M 1/00, A61F 9/007, A61M 3/02

(54) **VORRICHTUNG ZUR STEUERUNG EINES OPHTHALMOLOGISCHEN SYSTEMS SOWIE EIN OPHTHALMOLOGISCHES SYSTEM**
DEVICE FOR CONTROLLING AN OPHTHALMOLOGICAL SYSTEM, AND OPHTHALMOLOGICAL SYSTEM
DISPOSITIF POUR LA COMMANDE D'UN SYSTÈME OPHTALMOLOGIQUE AINSI QUE SYSTÈME OPHTALMOLOGIQUE

(30) Priorität: 27.07.2017 DE 102017213007
(43) Veröffentlichungstag der Anmeldung: 15.05.2019
(73) Patentinhaber: Geuder AG, 69126 Heidelberg (DE)
(72) Erfinder: MONTAG, Bernd, 69198 Schriesheim (DE)
(74) Vertreter: Ullrich & Naumann PartG mbB
(86) Internationale Anmeldenummer: PCT/DE2018/200068
(87) Internationale Veröffentlichungsnummer: WO 2019/020150

(56) Entgegenhaltungen:
- DE-A1- 102015 003 799
- US-A- 4 468 219
- US-A1- 2002 019 607
- US-A1- 2006 122 556
- US-A1- 2010 280 434
- US-A1- 2016 220 751

## Beschreibung

Die vorliegende Erfindung betrifft ein (nicht beanspruchtes) Verfahren zur Steuerung eines ophthalmologischen Systems, insbesondere eines Phakoemulsifikationssystems. Des Weiteren betrifft die Erfindung ein ophthalmologisches System, insbesondere ein Phakoemulsifikationssystem, vorzugsweise zu Durchführung des Verfahrens Ein System aus dem Stand der Technik ist aus DE 10 2015 003799 A1 bekannt.

Ophthalmologische Systeme und Verfahren zu deren Steuerung sind bereits seit Jahren aus der Praxis bekannt. Bei einem solchen System kann es sich beispielsweise um ein Phakoemulsifikationssystem handeln. Dieses besteht aus einer Konsole, an die ein Handstück anschließbar ist. Über die Konsole wird dem Handstück insbesondere eine Irrigationsflüssigkeit, Unterdruck sowie elektrische Leistung zu Verfügung gestellt. Im Rahmen einer Kataraktoperation, bei der die getrübte Linse eines Patienten aus dem Auge zu entfernen ist, wird eine von dem Handstück getragene Nadel in das Auge eingeführt und mit einer Ultraschallschwingung beaufschlagt. Durch die Schwingung wird die Linse zerstört bzw. emulsifiziert. Die Linsentrümmer werden durch die Nadel und das Handstück aus dem Auge abgesaugt - aspiriert - wozu von der Konsole ein Unterdruck erzeugt wird.

Um das Auge vor Verletzungen zu schützen, muss der Kammerdruck während des Eingriffs aufrecht erhalten werden. Hierzu wird dem Auge über die Konsole und das Handstück eine Irrigationsflüssigkeit zugeleitet, wodurch der Kammerdruck gezielt auf dem notwendigen Niveau gehalten wird. Dabei ist wesentlich, dass dem Auge weder zu viel noch zu wenig Irrigationsflüssigkeit zugeführt wird. Folglich ist eine Balance zu halten zwischen Irrigation und Aspiration. Dazu ist es bekannt, über Durchflusssensoren die zugeführte Irrigationsflüssigkeit sowie die aspirierte Flüssigkeit zu detektieren, um anhand der gewonnenen Informationen den Irrigationsdruck und den Aspirationsdruck aufeinander abzustimmen. Da ein in Rede stehender Eingriff am Auge unter sterilen Bedingungen zu erfolgen hat, werden besondere Herausforderungen an die verwendeten Flusssensoren gestellt und sind diese kompliziert aufgebaut.

Die aus dem Stand der Technik bekannten ophthalmologischen Systeme und Verfahren zum Betreiben dieser Systeme ermöglichen zwar, dass der Kammerdruck im Auge während eines Eingriffs aufrecht erhalten wird, sind in Konstruktion, Funktionsweise und Bedienung jedoch äußerst aufwändig.

Der vorliegenden Erfindung liegt daher die Aufgabe zugrunde, ein solches System derart auszugestalten und weiterzubilden, dass mit einfachen Mitteln ein stabiler Kammerdruck während eines Eingriffs gewährleistet ist.

Erfindungsgemäß wird die voranstehende Aufgabe durch die Merkmale des Anspruches 1 gelöst. Außerdem ist ein Verfahren zur Steuerung eines ophthalmologischen Systems, insbesondere eines Phakoemulsifikationssystems, offenbart, umfassend eine Steuereinheit, eine Irrigationseinheit und eine Aspirationseinheit, wobei die Irrigationseinheit eine Irrigationsperistaltikpumpe mit einem Irrigationsantrieb und die Aspirationseinheit eine Aspirationsperistaltikpumpe mit einem Aspirationsantrieb aufweisen und wobei der Irrigationsantrieb mit einer zumindest geringfügig höheren Drehzahl betrieben wird als der Aspirationsantrieb.

In erfindungsgemäßer Weise ist zunächst erkannt worden, dass die zugrunde liegende Aufgabe auf einfache Weise gelöst werden kann, wenn die Irrigation und die Aspiration über, identische, Peristaltikpumpen erfolgt, die insbesondere zumindest im Wesentlichen identische Schläuche aufweisen. Der Erfindung liegt dabei die Idee zu Grunde, dass wenn diese Peristaltikpumpen mit identischer Drehzahl betrieben werden, die Durchflussrate ebenfalls identisch ist. Somit werden gleiche Volumina gefördert werden, so dass eine Balance zwischen Irrigation und Aspiration besteht. In weiter erfindungsgemäßer Weise ist dabei erkannt worden, dass ein sicheres intraoperatives Szenario erzielbar ist, wenn die Drehzahl der Irrigationspumpe zumindest geringfügig über der Drehzahl der Aspirationspumpe liegt. Bei einer solchen Steuerung wird das Auge mit einem Irrigationsüberdruck stetig tonisiert und wird des Weiteren eine stets auftretende Leckage von Flüssigkeit aus der Inzision des Auges ausgeglichen. Somit ist auf einfache Weise ein stabiler Kammerdruck während des Eingriffs gewährleistet.

In vorteilhafter Weise wird als Irrigationsantrieb und als Aspirationsantrieb jeweils ein Schrittmotor verwendet. Die Schrittmotoren können in besonders vorteilhafter Weise im Teilschrittbetrieb betrieben werden, wobei Beschleunigung und Abbremsen im Rampenbetrieb erfolgen können. Beispielsweise kann der Motor derart ausgebildet sein, dass 200 Vollschritte pro Umdrehung notwendig sind, wobei ein Vollschritt in 8 Teilschritte auflösbar ist. Somit wären 1600 Vollschritte für eine Umdrehung notwendig. Generell sind feinere oder gröbere Auflösungen denkbar.

In weiter vorteilhafter Weise kann eine Irrigationsdrehzahl des Irrigationsantriebs in Abhängigkeit von der Höhe, auf der sich ein Irrigationsbehälter befindet, bestimmt werden. Somit werden der durch die Höhe des Behälters bereits anfallende Irrigationsdruck und der damit einhergehende Irrigationsfluss berücksichtigt. Alternativ oder zusätzlich ist es denkbar, dass in Abhängigkeit eines gewählten Aspirationsunterdrucks und/oder eines gewählten Aspirationsdurchflusses und/oder eines gemessenen Aspirationsunterdrucks eine Aspirationsdrehzahl des Aspirationsantriebs bestimmt wird. Beispielsweise kann der Operateur über ein Interface einen Aspirationsunterdruck bzw. einen Aspirationsdurchfluss auswählen.

In besonders vorteilhafter Weise wird anhand der Irrigationsdrehzahl und anhand der Aspirationsdrehzahl bestimmt, um welchen Differenzbetrag die tatsächliche Irrigationsdrehzahl über der tatsächlichen Aspirationsdrehzahl zu liegen hat. Durch eine solche Bestimmung bzw. Berechnung des Differenzbetrag wird somit festgelegt, wieviel höher die Drehzahl des Irrigationsantriebs als die Drehzahl des Aspirationsantriebs ist.

Um einen sicheren Betrieb des Systems zu ermöglichen, können während des Betriebs die tatsächliche Drehzahl des Irrigationsantriebs und die tatsächliche Drehzahl des Aspirationsantriebs erfasst werden. Zusätzlich ist es denkbar, dass die Drehzahl des Irrigationsantriebs erhöht wird, wenn die erfasste tatsächliche Drehzahl des Irrigationsantriebs nicht mindestens um den Differenzbetrag höher ist als die tatsächliche Drehzahl des Aspirationsantriebs. Ebenso kann es möglich sein, die Drehzahl des Aspirationsantriebs in einem solchen Fall zu verringern. Wesentlich ist, dass eine Überwachung der Drehzahlen von Irrigationsantrieb und Aspirationsantrieb erfolgt, um nämlich sicher zu stellen, dass die Drehzahl des Irrigationsantriebs höher ist als die Drehzahl des Aspirationsantriebs.

Die zugrunde liegende Aufgabe wird durch die Merkmale des Anspruchs 1 gelöst. Damit ist ein ophthalmologisches System angegeben, insbesondere ein Phakoemulsifikationssystem, mit einer Steuereinheit, einer Irrigationseinheit umfassend eine Irrigationsperistaltikpumpe mit einem Irrigationsantrieb und einer Aspirationseinheit umfassend eine Aspirationsperistaltikpumpe mit einem Aspirationsantrieb, wobei der Aspirationsantrieb und der Irrigationsantrieb mittels der Steuereinheit derart steuerbar sind, dass die Drehzahl des Irrigationsantriebs zumindest geringfügig höher ist als die Drehzahl des Aspirationsantriebs.

In erfindungsgemäßer Weise ist dabei erkannt worden, dass die zugrunde liegende Aufgabe gelöst werden kann, indem die Irrigationseinheit und die Aspirationseinheit jeweils eine identische Peristaltikpumpe, insbesondere mit identischen Schläuchen aufweisen. Bei einer solchen Ausgestaltung können durch eine Steuereinheit auf einfache Weise die Drehzahl des Irrigationsantriebs und die Drehzahl des Aspirationsantriebs derart steuerbar sein, dass der Irrigationsantrieb mit einer zumindest geringfügig höheren Drehzahl als der Aspirationsantrieb betrieben wird. Bei einer solchen Ausgestaltung liegt der Irrigationsdruck stets über dem Aspirationsdruck, so dass Leckageverluste ausgeglichen und das Auge tonisiert wird.

In vorteilhafter Weise können der Irrigationsantrieb und der Aspirationsantrieb jeweils als Schrittmotor ausgebildet sein. Bei einer solchen Konstruktion ist eine besonders genaue Einstellung der Drehzahlen möglich.

An dieser Stelle wird darauf hingewiesen, dass das offenbarte Verfahren auch eine vorrichtungsmäßige Ausprägung hat, so dass diese Verfahrensmerkmale ausdrücklich Teil der Offenbarung des erfindungsgemäßen ophthalmologischen Systems gemäß Anspruch 1 sind.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigt
- Fig. 1: in einer schematischen Prinzipskizze ein Ausführungsbeispiel eines erfindungsgemäßen Systems, an dem auch das erfindungsgemäße Verfahren erläutert wird, und
- Fig. 2: an Ablaufdiagramm eines Ausführungsbeispiels eines erfindungsgemäßen Verfahrens.

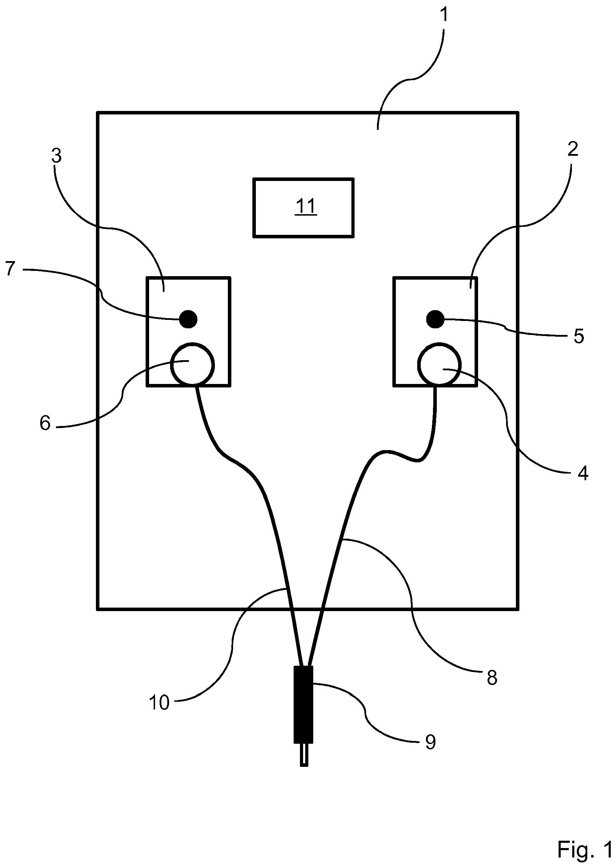

Fig. 1 zeigt eine Prinzipskizze eines ophthalmologischen Systems. Das System umfasst eine Konsole 1 mit einer Irrigationseinheit 2 und einer Aspirationseinheit 3. Die Irrigationseinheit 2 weist eine Irrigationsperistaltikpumpe 4 und einen Irrigationsantrieb 5 auf. Des Weiteren umfasst die Aspirationseinheit 3 eine Aspirationsperistaltikpumpe 6 und einen Aspirationsantrieb 7.

Über einen Irrigationsschlauch 8 wird Irrigationsflüssigkeit einem Handstück 9 zugeführt und über einen Aspirationsschlauch 10 Flüssigkeit und ggf. Gewebetrümmer von dem Handstück 9 abgesaugt. Es wird darauf hingewiesen, dass die Irrigationseinheit 2 und/oder die Aspirationseinheit 3 nicht zwangsweise in einer Konsole 1 bzw. in der gleichen Konsole 1 angeordnet sein müssen.

Des Weiteren ist eine Steuereinheit 11 vorgesehen, über die die Irrigationseinheit 2 und die Aspirationseinheit 3, insbesondere die Drehzahl der Irrigationspumpe 4 und die Drehzahl der Aspirationspumpe 6 steuerbar ist. Erfindungsgemäß sind die Irrigationspumpe 4 und die Aspirationspumpe 6 identisch ausgebildet, ebenso wie der Irrigationsantrieb 5 und der Aspirationsantrieb 7.

Die Drehzahl des Irrigationsantriebs 5 wird dabei derart gesteuert, dass sie zumindest geringfügig über der Drehzahl des Aspirationsantriebs 7 liegt. Dadurch wird sicher gestellt, dass das Auge tonisiert und Leckage ausgeglichen wird. Die Irrigationsflüssigkeit kann in einem nicht dargestellten Irrigationsbehälter vorgehalten sein, der beispielsweise in der Höhe verfahrbar sein kann.

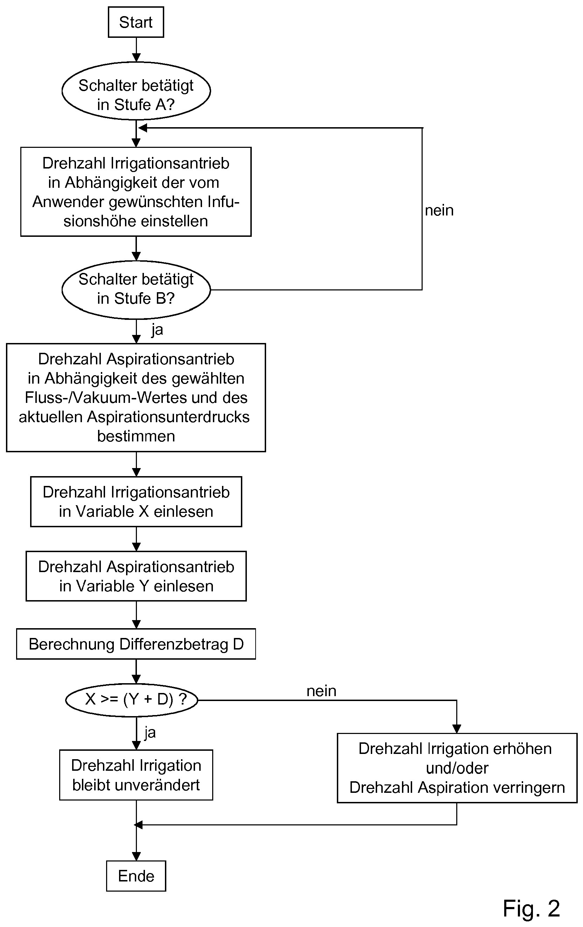

Fig. 2 zeigt ein Ablaufdiagramm eines erfindungsgemäßen Verfahrens, insbesondere zur Steuerung einer erfindungsgemäßen Vorrichtung gemäß Fig. 1. Nach dem Starten des ophthalmologischen Systems wird zunächst überprüft, ob der Operateur oder eine Hilfskraft einen Schalter betätigt hat. Hierbei kann es sich beispielsweise um einen Fußschalter handeln, der in Stufe A gestellt werden muss, um das Verfahren zu beginnen. Die Irrigationsdrehzahl wird sodann in Abhängigkeit einer von dem Anwender gewählten Höhe des Irrigationsbehälters eingestellt bzw. bestimmt.

Sofern der Schalter daraufhin in Stufe B gestellt wird, wird die Aspirationsdrehzahl in Abhängigkeit eines von dem Anwender gewählten Durchfluss- und/oder Vakuumwertes und/oder des aktuellen, gemessenen Aspirationsunterdrucks berechnet bzw. bestimmt. Die berechneten bzw. bestimmten Werte der Irrigationsdrehzahl und der Aspirationsdrehzahl werden als Variablen X und Y eingelesen und auf dieser Grundlage der Differenzbetrag D bestimmt.

Während der Verwendung des Systems wird stets überprüft, ob die Irrigationsdrehzahl X mindestens um den Differenzbetrag D über der Aspirationsdrehzahl Y liegt. Ist dies der Fall, bleiben die Irrigationsdrehzahl und die Aspirationsdrehzahl unverändert. Ansonsten wird die Irrigationsdrehzahl erhöht und/oder die Aspirationsdrehzahl verringert.

Hinsichtlich weiterer vorteilhafter Ausgestaltungen des erfindungsgemäßen Verfahrens und der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele des offenbarten Verfahrens und der erfindungsgemäßen Vorrichtung lediglich zur Erörterung der beanspruchten Lehre dienen, diese jedoch nicht auf die Ausführungsbeispiele einschränken. Die Erfindung wird durch die Ansprüche definiert.

### Bezugszeichenliste

- 1: Konsole
- 2: Irrigationseinheit
- 3: Aspirationseinheit
- 4: Irrigationsperistaltikpumpe
- 5: Irrigationsantrieb
- 6: Aspirationsperistaltikpumpe
- 7: Aspirationsantrieb
- 8: Irrigationsschlach
- 9: Handstück
- 10: Aspirationsschlauch
- 11: Steuereinheit

## Patentansprüche

1. Ophthalmologisches System, insbesondere Phakoemulsifikationssystem, mit einer Steuereinheit (11), einer Irrigationseinheit (2) umfassend eine Irrigationsperistaltikpumpe (4) mit einem Irrigationsantrieb (5) und einer Aspirationseinheit (3) umfassend eine Aspirationsperistaltikpumpe (6) mit einem Aspirationsantrieb (7), wobei der Aspirationsantrieb (7) und der Irrigationsantrieb (5) mittels der Steuereinheit (11) derart steuerbar sind, dass die Drehzahl des Irrigationsantriebs (5) zumindest geringfügig größer ist als Drehzahl des Aspirationsantriebs (7) und wobei die Irrigationsperistaltikpumpe (4) und die Aspirationsperistaltikpumpe (6) identisch ausgebildet sind.

2. System nach Anspruch 1, **dadurch gekennzeichnet, dass** der Irrigationsantrieb (5) und der Aspirationsantrieb (7) jeweils als Schrittmotor ausgestaltet sind.

3. System nach Anspruch 1 oder 2, **dadurch gekennzeichnet, dass** in Abhängigkeit von der Höhe, auf der sich ein Irrigationsbehälter befindet, eine Irrigationsdrehzahl des Irrigationsantriebs (5) bestimmbar ist.

4. System nach einem der Ansprüche 1 bis 3, **dadurch gekennzeichnet, dass** in Abhängigkeit eines gewählten Aspirationsunterdrucks und/oder Aspirationsdurchflusses eine Aspirationsdrehzahl des Aspirationsantriebs (7) bestimmbar ist.

5. System nach einem der Ansprüche 1 bis 4, **dadurch gekennzeichnet, dass** in Abhängigkeit eines gemessenen Aspirationsunterdrucks eine Aspirationsdrehzahl des Aspirationsantriebs (7) bestimmbar ist.

6. System nach einem der Ansprüche 3 bis 5, **dadurch gekennzeichnet, dass** anhand der Irrigationsdrehzahl und der Aspirationsdrehzahl bestimmbar ist, um welchen Differenzbetrag D die tatsächliche Irrigationsdrehzahl über der tatsächlichen Aspirationsdrehzahl zu liegen hat.

7. System nach einem der Ansprüche 1 bis 6, **dadurch gekennzeichnet, dass** während des Betriebs die tatsächliche Drehzahl des Irrigationsantriebs (5) und die tatsächliche Drehzahl des Aspirationsantriebs (7) erfassbar sind.

8. System nach Anspruch 7, **dadurch gekennzeichnet, dass** die Drehzahl des Irrigationsantriebs (5) erhöhbar ist, wenn die tatsächliche Drehzahl des Irrigationsantriebs (5) nicht mindestens um den Differenzbetrag D höher ist als die tatsächliche Drehzahl des Aspirationsantriebs (7).

9. System nach Anspruch 7 oder 8, **dadurch gekennzeichnet, dass** die Drehzahl des Aspirationsantriebs (7) verringerbar ist, wenn die tatsächliche Drehzahl des Irrigationsantriebs (5) nicht mindestens um den Differenzbetrag D höher ist als die tatsächliche Drehzahl des Aspirationsantriebs (7).

## Claims

1. Ophthalmological system, in particular phacoemulsification system, having a control unit (11), an irrigation unit (2) comprising a peristaltic irrigation pump (4) having an irrigation drive (5) and an aspiration unit (3) comprising a peristaltic aspiration pump (6) having an aspiration drive (7), wherein the aspiration drive (7) and the irrigation drive (5) can be controlled by means of the control unit (11) in such a manner that the speed of the irrigation drive (5) is at least slightly greater than the speed of the aspiration drive (7) and wherein the peristaltic irrigation pump (4) and the peristaltic aspiration pump (6) are constructed in an identical manner.

2. System according to claim 1, **characterised in that** the irrigation drive (5) and the aspiration drive (7) are in each case in the form of a step motor.

3. System according to claim 1 or 2, **characterised in that**, depending on the height at which an irrigation container is located, an irrigation speed of the irrigation drive (5) can be determined.

4. System according to any one of claims 1 to 3, **characterised in that**, depending on a selected reduced aspiration pressure and/or aspiration throughflow, an aspiration speed of the aspiration drive (7) can be determined.

5. System according to any one of claims 1 to 4, **characterised in that**, depending on a measured reduced aspiration pressure, an aspiration speed of the aspiration drive (7) can be determined.

6. System according to any one of claims 3 to 5, **characterised in that** with reference to the irrigation speed and the aspiration speed it is possible to determine the difference amount D by which the actual irrigation speed has to be above the actual aspiration speed.

7. System according to any one of claims 1 to 6, **characterised in that** during operation the actual speed of the irrigation drive (5) and the actual speed of the aspiration drive (7) can be detected.

8. System according to claim 7, **characterised in that** the speed of the irrigation drive (5) can be increased when the actual speed of the irrigation drive (5) is not higher than the actual speed of the aspiration drive (7) by at least the difference amount D.

9. System according to claim 7 or 8, **characterised in that** the speed of the aspiration drive (7) can be decreased when the actual speed of the irrigation drive (5) is not higher than the actual speed of the aspiration drive (7) by at least the difference amount D.

## Revendications

1. Système ophtalmologique, en particulier système de phacoémulsification, avec une unité de commande (11), une unité d'irrigation (2) comprenant une pompe péristaltique d'irrigation (4) avec un entraînement d'irrigation (5) et une unité d'aspiration (3) comprenant une pompe péristaltique d'aspiration (6) avec un entraînement d'aspiration (7), dans lequel l'entraînement d'aspiration (7) et l'entraînement d'irrigation (5) peuvent être commandés par l'unité de commande (11) de telle sorte que la vitesse de rotation de l'entraînement d'irrigation (5) est au moins légèrement supérieure à la vitesse de rotation de l'entraînement d'aspiration (7), et dans lequel la pompe péristaltique d'irrigation (4) et la pompe péristaltique d'aspiration (6) sont conçues de manière identique.

2. Système selon la revendication 1, **caractérisé en ce que** l'entraînement d'irrigation (5) et l'entraînement d'aspiration (7) sont chacun conçus comme un moteur pas à pas.

3. Système selon la revendication 1 ou 2, **caractérisé en ce qu'**une vitesse de rotation d'irrigation de l'entraînement d'irrigation (5) peut être déterminée en fonction de la hauteur à laquelle se trouve un réservoir d'irrigation.

4. Système selon l'une des revendications 1 à 3, **caractérisé en ce qu'**une vitesse de rotation d'aspiration de l'entraînement d'aspiration (7) peut être déterminée en fonction d'une sous-pression d'aspiration et/ou d'un débit d'aspiration choisi(e).

5. Système selon l'une des revendications 1 à 4, **caractérisé en ce qu'**une vitesse de rotation d'aspiration de l'entraînement d'aspiration (7) peut être déterminée en fonction d'une sous-pression d'aspiration mesurée.

6. Système selon l'une des revendications 3 à 5, **caractérisé en ce qu'**il est possible de déterminer, à partir de la vitesse de rotation d'irrigation et de la vitesse de rotation d'aspiration, de quelle valeur différentielle D la vitesse de rotation d'irrigation réelle doit être supérieure à la vitesse de rotation d'aspiration réelle.

7. Système selon l'une des revendications 1 à 6, **caractérisé en ce que**, pendant le fonctionnement, la vitesse de rotation réelle de l'entraînement d'irrigation (5) et la vitesse de rotation réelle de l'entraînement d'aspiration (7) peuvent être détectées.

8. Système selon la revendication 7, **caractérisé en ce que** la vitesse de rotation de l'entraînement d'irrigation (5) peut être augmentée si la vitesse de rotation réelle de l'entraînement d'irrigation (5) n'est pas supérieure d'au moins la valeur différentielle D à la vitesse de rotation réelle de l'entraînement d'aspiration (7).

9. Système selon la revendication 7 ou 8, **caractérisé en ce que** la vitesse de rotation de l'entraînement d'aspiration (7) peut être réduite si la vitesse de rotation réelle de l'entraînement d'irrigation (5) n'est pas supérieure d'au moins la valeur différentielle D à la vitesse de rotation réelle de l'entraînement d'aspiration (7).
